# FASCICULE DE BREVET EUROPEEN

(11) **EP 1 212 119 B1**
(45) Date de publication et mention de la délivrance du brevet: **24.03.2004**
(21) Numéro de dépôt: 00956565.6
(22) Date de dépôt: 28.07.2000
(51) Int. Cl.: A61P 25/30, A61K 31/5415

(54) **UTILISATION DE LA CYAMEMAZINE DANS LE TRAITEMENT DU SEVRAGE BRUTAL AUX BENZODIAZEPINES**
VERWENDUNG VON CYAMEMAZIN ZUR BEHANDLUNG VON ENTWÖHNUNGSERSCHEINUNGEN DER BENZODIAZEPINBEHANDLUNG
USE OF CYAMEMAZINE FOR the treatment of abrupt benzodiazepine withdrawal

(30) Priorité: 13.08.1999 FR 9910472
(43) Date de publication de la demande: 12.06.2002
(73) Titulaire: Aventis Pharma S.A., 92160 Antony (FR)
(72) Inventeur: D'ALCHE-BIREE, Françoise, F-75013 Paris (FR)
(74) Mandataire: Rousseau, Pierrick Edouard
(86) Numéro de dépôt international: PCT/FR2000/002186
(87) Numéro de publication internationale: WO 2001/012265

(56) Documents cités:
- EP-A- 0 429 360
- WO-A-98/43646
- US-A- 2 877 224
- PARQUET P.-J. ET AL: "[Drugs used in the treatment of psychoactive substances withdrawal]. LES MEDICAMENTS DU SEVRAGE DES TOXICOMANIES." PSYCHOLOGIE MEDICALE, (1989) 21/13 (2012-2016)., XP000908865
- NAASSILA, MICKAEL ET AL: "Cyamemazine decreases ethanol intake in rats and convulsions during ethanol withdrawal syndrome in mice." PSYCHOPHARMACOLOGY, (DEC., 1998) VOL. 140, NO. 4, PP. 421-428., XP000908904
- E.F. REYNOLDS: "Martindale (Thirty-First Edition)" 1996 , ROYAL PHARMACEUTICAL SOCIETY , LONDON XP002137444 page 699, colonne 3

## Description

La présente invention concerne l'utilisation de la cyamémazine ou ses sels pharmaceutiquement acceptables dans le traitement du sevrage brutal aux benzodiazépines par substitution de la benzodiazépine par la cyamémazine.

Les benzodiazépines telles que bromazépam, oxazépam, lorazépam, alprozolam, diazépam, prazépam, nordazépam, clobazam, clotiazépam, aprazolam sont le traitement médicamenteux de référence des troubles anxieux. Cependant la survenue d'une dépendance psychologique et/ou physique a été décrite lors de prescriptions supérieures à 6 semaines (KG. POWER et coll., Br. Med. J., 290, 1246 (1985)). La fréquence de survenue du syndrome de sevrage après arrêt des benzodiazépines varie selon les critères choisis mais est généralement de l'ordre de 40% (R. NOYES et coll., J. Clin. Psychiatry, 49, 382 (1988)).

Il est donc recommandé, d'une part, de limiter la prescription des benzodiazépines à 3 mois, renouvelables dans certains cas d'anxiété sévère et, d'autre part, de procéder à une arrêt progressif du traitement. Toutefois, même en cas d arrêt progressif, on peut noter la survenue d'un syndrome de sevrage, responsable d'une reprise du traitement par benzodiazépines (E. SCHWEIZER et coll., Arch. Gen. Psychiatry, 47, 908 (1990)).

La cyamémazine (TERCIAN^{(R)}) est un neuroleptique sédatif ayant, à faible posologie, une bonne tolérance. C'est un traitement de l'anxiété indiqué dans les cas d'inefficacité des thérapeutiques habituelles ou dans les cas d'anxiétés névrotiques ou psychotiques. Son action sur la diminution de prise d'alcool chez le rat est également connue (N. NAASILA et coll., Psychopharmacology, 140, 421 (1998)).

Des anxiolytiques non benzodiazépiniques comme la buspirone ont été testés dans le sevrage aux benzodiazépines mais n'ont ont pas montré d'effet significatif sur le syndrome de sevrage (LADER et coll., Journal of clinical Psychopharmacology, 7, 1 (1987) ; SCHWEIZER et coll., Acta Psychiatr Scandinavia, 98, sup 393 (1998).

Il a maintenant été trouvé que la cyamémazine permet de traiter les symptômes et notamment le rebond d'anxiété et le syndrome de sevrage, survenant à l'arrêt du traitement des benzodiazépines et éviter ainsi la reprise des benzodiazépines et la pérénisation du traitement. Cet effet est toujours constaté 6 mois après le traitement.

Au cours d'un essai clinique, ont été comparées 2 stratégies d'arrêt des benzodiazépines : soit un sevrage progressif par une benzodiazépine (bromazépam), soit un sevrage brutal et une substitution par la cyamémazine.

La cyamémazine a été étudiée chez 168 patients âgés de 18 à 65 ans, traités par des benzodiazépines (bromazépam, oxazépam, lorazépam ou alprazolam) depuis 3 mois ou plus, à une posologie supérieure ou égale à 5 mg équivalent-diazépam et inférieure à 20 mg équivalent-diazépam depuis au moins 15 jours, présentant un score à l'échelle d'anxiété de HAMILTON (Br. J. Med. Psychol., 32, 50 (1959)) inférieure à 18 et nécessitant un sevrage aux benzodiazépines.

### Tableau d'équivalence diazépam :

| PRODUITS | POSOLOGIE (mg) |
|---|---|
| diazépam | 10 |
| alprazolam | 1 |
| bromazépam | 6 |
| lorazépam | 2,5 |
| oxazépam | 50 |

Le traitement est randomisé en double insu et se déroule sur 3 périodes :

### 1 - période de substitution

La benzodiazépine est remplacée soit par la cyamémazine soit par le bromazépam.

La posologie est fixée d'après le traitement benzodiazépinique antérieur :
- elle est de 4 gélules, en 2 ou 3 prises par jour, dosées à 12,5 mg de cyamémazine ou 1,5 mg de bromazépam, lorsque la posologie antérieure de la benzodiazépine est égale ou supérieure à 10 mg équivalent-diazépam et inférieure à 20 mg équivalent-diazépam,
- elle est de 2 gélules, en 2 prises par jour, dosées à 12,5 mg de cyamémazine ou 1,5 mg de bromazépam lorsque la posologie antérieure de la benzodiazépine est égale ou supérieure à 5 mg équivalent-diazépam et inférieure à 10 mg équivalent-diazépam,

### 2 - période de diminution de la posologie

la posologie est soit 1 ou 2 gélules à 12,5 mg de cyamémazine ou à 1,5 mg de bromazépam par jour pendant 14 jours, soit 1 ou 2 gélules à 12,5 mg de cyamémazine ou à 1,5 mg de bromazépam par jour pendant 11 jours puis 3 jours de placébo

### 3 - période d'arrêt

1 ou 2 gélules par jour de placébo pendant 14 jours.

Les résultats sont déterminés pour chaque malade selon les critères suivants :
■ échelle d'anxiété de HAMILTON (HARS)
■ autoévaluation d'anxiété de ZUNG (Official Journal of the Academy of psychosomatic Medicine, vol XII, n°6 (1971))
■ échelle de sevrage de RICKELS (Arch. Gen. Psychiatry, vol 47 (1990))

Les résultats obtenus dans chacun de ces tests permettent de déduire qu'il n'y a pas de différence significative entre le groupe de traitement cyamémazine et le groupe de traitement bromazépam, sur l'amplitude maximale du rebond d'anxiété (échelle HARS et échelle d'anxiété de ZUNG) et le pourcentage de patients présentant un rebond d'anxiété. L'échelle de sevrage de RICKELS ne montre également pas de différence significative entre les 2 groupes de traitement sur la fréquence de survenue d'un syndrome de sevrage. Cependant, après 6 semaines d'étude, le traitement est un succès chez un plus grand nombre de patients du groupe cyamémazine (95%) que chez les patients du groupe bromazépam (85%). Par ailleurs, à distance du sevrage (6 mois), le sevrage est toujours un succès chez 90% des patients sous cyamémazine versus 75% sous bromazépam.

Ces résultats démontrent que lorsqu'il est nécessaire d'arrêter un traitement chronique par benzodiazépines, la cyamémazine peut être utilisée par substitution brutale à la benzodiazépine sans conséquences liées à la survenue d'un rebond d'anxiété ou d'un syndrome de sevrage. Par ailleurs, la substitution par la cyamémazine pendant 6 semaines est suivi d'un meilleur taux de succès du sevrage à distance (6 mois).

La cyamémazine peut être préparée selon le brevet américain 2877224.

Comme sels pharmaceutiquement acceptables de la cyamémazine peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, isethionate, théophilline-acétate, salicylate, phénolphtalinate, méthylène-bis-β-oxynaphtoate ou des dérivés de substitution de ces dérivés.

Les médicaments sont constitués par au moins la cyamémazine sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 10 et 100 mg par jour et, en particulier 25 à 100 mg par voie orale pour un adulte avec des doses unitaires allant de 10 à 20 mg de substance active.

D'une façon générale, le médecin déterminera la posologie appropriée en fonction de l'âge, du poids et de tous les autres facteurs propres au sujet à traiter.

Les exemples suivants illustrent des médicaments selon l'invention :

### Exemple A

On prépare, selon la technique habituelle, des comprimés dosés à 20 mg de produit actif ayant la composition suivante :
- Cyamémazine 20 mg
- Mannitol 64 mg
- Cellulose microcristalline 50 mg
- Polyvidone excipient 12 mg
- Carboxyméthylamidon sodique 16 mg
- Talc 4 mg
- Stéarate de magnésium 2 mg
- Silice colloïdale anhydre 2 mg
- Mélange de méthylhydroxypropylcellulose,
   polyéthylèneglycol 6000, dioxyde de titane (72-3,5-24,5)
   q.s.p. 1 comprimé pelliculé terminé à 245 mg

### Exemple B

On prépare, selon la technique habituelle, des gélules dosées à 20 mg de produit actif ayant la composition suivante :
- cyamémazine 20 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### Exemple C

On prépare une solution injectable contenant 10 mg de produit actif ayant la composition suivante :
- Cyamémazine 10 mg
- Acide benzoïque 80 mg
- Alcool benzylique 0,06 cm3
- Benzoate de sodium 80 mg
- Ethanol à 95 % 0,4 cm3
- Hydroxyde de sodium 24 mg
- Propylène glycol 1,6 cm3
- Eau q.s.p. 4 cm3

L'invention concerne également l'utilisation de la cyamémazine ou un de ses sels pharmaceutiquement acceptables pour la préparation d'un médicament utile pour le sevrage brutal aux benzodiazépines par substitution de la benzodiazépine par la cyamémazine.

L'invention concerne également le procédé de préparation de médicaments utiles dans le sevrage brutal aux benzodizépines par substitution de la benzodizépine par la cyamémazine consistant à mélanger la cyamémazine ou les sels pharmaceutiquement acceptables de ce composé avec un ou plusieurs diluants et/ou adjuvants compatibles et pharmaceutiquement acceptables.

## Revendications

1. Utilisation de la cyamémazine ou un de ses sels pharmaceutiquement acceptables pour la préparation d'un médicament destiné au traitement du sevrage brutal aux benzodiazépines par substitution de la benzodiazépine par la cyamémazine.

2. Utilisation selon la revendication 1 pour la préparation d'un médicament comprenant 10 à 20 mg de cyamémazine.

## Patentansprüche

1. Verwendung von Cyamemazin oder einem seiner pharmazeutisch annehmbaren Salze für die Herstellung eines Medikaments, das für die Behandlung beim plötzlichen Absetzen von Benzodiazepinen durch Substitution des Benzodiazepins durch Cyamemazin bestimmt ist.

2. Verwendung gemäß Anspruch 1 für die Herstellung eines Medikaments, das 10 bis 20 mg Cyamemazin enthält.

## Claims

1. Use of cyamemazine, or a pharmaceutically acceptable salt thereof, for preparing a medicinal product intended for the treatment of abrupt benzodiazepine withdrawal by substitution of the benzodiazepine with cyamemazine.

2. Use according to Claim 1, for preparing a medicinal product comprising 10 to 20 mg of cyamemazine.
